# FASCICULE DE BREVET EUROPEEN

(11) **EP 3 056 506 B1**
(45) Date de publication et mention de la délivrance du brevet: **26.08.2020**
(21) Numéro de dépôt: 16155976.0
(22) Date de dépôt: 16.02.2016
(51) Int. Cl.: C07H 1/00, C07H 3/06, A23L 33/21

(54) **MALTO-OLIGO-SACCHARIDES RICHES EN FIBRES ET PRÉSENTANT UNE FAIBLE BIOSDISPONIBILITÉ EN GLUCOSE, LEUR PROCÉDÉ DE FABRICATION ET LEURS UTILISATIONS EN NUTRITION HUMAINE ET ANIMALE**
FASERREICHE MALTOOLIGOSACCHARIDE MIT GERINGER GLUKOSEBIOVERFÜGBARKEIT, VERFAHREN ZU IHRER HERSTELLUNG UND DEREN VERWENDUNG ZUR MENSCHLICHEN UND TIERISCHEN ERNÄHRUNG
MALTOOLIGOSACCHARIDES HAVING A HIGH FIBER CONTENT AND A LOW GLUCOSE BIOAVAILABILITY, PROCESS FOR THE PREPARATION THEREOF AND USE THEREOF IN HUMAN AND ANIMAL NUTRITION

(30) Priorité: 16.02.2015 FR 1551274
(43) Date de publication de la demande: 17.08.2016
(73) Titulaire: Roquette Frères, 62136 Lestrem (FR)
(72) Inventeur: DUFLOT, Pierrick, 62136 La Couture (FR); ROTURIER, Jean-Michel, 59280 Armentières (FR); BOIT, Baptiste, 59253 La Gorgue (FR); LANOS, Pierre, 59480 La Bassée (FR); JEROSCH, Heike, 59940 Estaires (FR)
(74) Mandataire: Plasseraud IP

(56) Documents cités:
- EP-A1- 1 006 128
- EP-A2- 0 675 137
- WO-A1-2013/128121

## Description

### DOMAINE DE L'INVENTION

La présente invention se rapporte selon un premier mode à des malto-oligo-saccharides dont la teneur en liaisons osidiques α 1-4 est comprise entre 70 % et 80 % du nombre total de liaisons osidiques de type 1-4 et une teneur en liaisons α 1-6 comprise entre 35 et 58% du nombre total de liaisons osidiques 1-6. Le terme « malto-oligo-saccharides» fait ici référence à des saccharides comprenant au moins 2 unités saccharides, c'est-à-dire par exemple à des saccharides présentant un degré de polymérisation DP compris entre 2 et 30, lesdits saccharides comprenant au moins un glucide qui est le maltose. Le terme « polysaccharides », également utilisé dans la présente Demande, fait référence de manière plus large à des saccharides comprenant au moins 2 unités saccharides, c'est-à-dire par exemple à des saccharides présentant un degré de polymérisation DP compris entre 2 et 30.

De manière particulièrement avantageuse, c'est le réglage fin de la teneur en liaisons osidiques α 1-4 qui conduit à des produits uniques et conformes à la présente invention, attestant d'un compromis idéal entre une richesse en fibres relativement importante et une très faible biodisponibilité du glucose vis-à-vis de l'organisme. On dispose ainsi de produits mixtes et encore jamais identifiés, offrant tous les bénéfices des aliments à base de fibres, avec une valeur nutritionnelle extrêmement faible. Un tel compromis est particulièrement intéressant à mettre en œuvre dans des régimes alimentaires sains et équilibrés, mais aussi dans le traitement et/ou la prévention de la pathologie du diabète.

L'invention porte également sur un procédé original et très simple de mise en œuvre pour la fabrication de ces malto-oligo-saccharides, où un mélange entre une solution aqueuse de glucides riche en maltose, au moins un polyol et au moins un acide subit un traitement thermique à haute température (entre 130°C et 300°C) et sous pression réduite. Le choix des matières premières de départ et notamment de la solution aqueuse de glucides riche en maltose, mais aussi la durée du traitement font partie des leviers permettant de réguler la teneur en liaisons α 1-4 dans l'intervalle mentionné ci-dessus.

Un dernier objet de la présente invention consiste en l'utilisation des malto-oligo-saccharides selon l'invention, en nutrition humaine et animale.

### PROBLEME TECHNIQUE ET ART ANTERIEUR

Depuis plusieurs années, il existe un intérêt certain du grand public pour de nouveaux régimes alimentaires à base de fibres. Par fibres alimentaires, on entend les parties d'origine végétale qui ne sont pas hydrolysées par les enzymes au cours du phénomène de digestion. Ce sont des substances résiduelles provenant de la paroi cellulaire ou le cytoplasme des végétaux, constituées de mélanges complexes de glucides, qui ont été identifiés comme étant des polysaccharides non amylacés.

Parmi les fibres alimentaires, on distingue les fibres insolubles des fibres solubles dans l'eau. L'avoine, l'orge, les fruits, les légumes frais et les légumes secs (haricots, lentilles, pois chiches) constituent de bonnes sources de fibres solubles, tandis que les céréales complètes et le pain complet sont riches en fibres insolubles. Les fibres insolubles, comme la cellulose, les amidons résistants, les fibres de maïs (drêche) ou de soja, ont un rôle essentiellement mécanique dans le tractus gastro-intestinal. Elles ne sont que très peu fermentées par la flore colique et contribuent à la réduction du temps de transit intestinal par effet de lest. Les fibres insolubles contribuent ainsi à prévenir la constipation en augmentant le poids des selles et en réduisant la durée du transit intestinal.

Les fibres solubles, comme la pectine et l'inuline, non digestibles par les enzymes intestinales de l'homme ou de l'animal, sont fermentées par la flore colique. Cette fermentation libère des acides gras à courte chaîne dans le côlon, qui ont pour effet de diminuer le pH de celui-ci et par voie de conséquence de limiter le développement de bactéries pathogènes et de stimuler le développement des bactéries bénéfiques.

A côté de ces composés majoritairement extraits des plantes, existent des molécules dérivées de l'amidon ou de ses produits d'hydrolyse partielle ou totale. Le polydextrose est par exemple synthétisé par polymérisation aléatoire du glucose en présence de sorbitol et d'un catalyseur acide adéquat (tel l'acide citrique) et à haute température. Ledit polydextrose est largement utilisé dans l'alimentation comme agent de charge et comme ingrédient à faible caloricité.

De manière plus générale, les polymères de glucose sont classiquement fabriqués industriellement par hydrolyse des amidons naturels ou hybrides et de leurs dérivés. Ces hydrolysats d'amidon (dextrines, pyrodextrines, etc) sont ainsi produits par hydrolyse acide ou enzymatique d'amidon de céréales ou de tubercules. Ils sont en fait constitués d'un mélange de glucose et de polymères du glucose, de poids moléculaires très variés. Lesdits hydrolysats présentent une large distribution de saccharides contenant à la fois des structures linéaires (liaisons osidiques α 1-4) et branchées (liaisons osidiques α 1-6).

A titre d'exemples, les demandes de brevet EP 0 368 451 et US 5,264,568 décrivent un procédé de préparation de pyrodextrines, dont on renforce les caractéristiques de fibres alimentaires par action d'une α-amylase ou de plusieurs α-amylases successivement sur une dextrine ou sur une pyrodextrine en solution et à haute température.

Dans la demande de brevet EP 0 530 111, sont décrites des dextrines indigestibles obtenues par extrusion d'un amidon de maïs acidifié déshydraté dans des conditions particulières. Ce traitement peut être complété par l'action d'une α-amylase thermorésistante.

La société Demanderesse a elle-même également décrit dans sa demande de brevet EP 1 006 128 des maltodextrines branchées présentant entre 22 % et 35 % de liaisons osidiques 1-6 (à la fois de type α et β), une teneur en sucres réducteurs inférieure à 20 %, un indice de polymolécularité inférieur à 5 et une masse moléculaire moyenne en nombre Mn au plus égale à 4500 g/mole. Ces maltodextrines branchées présentent surtout un caractère d'indigestibilité qui a pour conséquence de diminuer leur pouvoir calorique, en empêchant leur assimilation au niveau de l'intestin grêle ; elles constituent donc essentiellement une source de fibres indigestibles.

La société Demanderesse a aussi décrit et protégé dans sa demande de brevet WO 2013/128121 des maltodextrines hyperbranchées de faible poids moléculaire, i.e. présentant un dextrose équivalent (DE) compris entre 8 et 15 et un poids moléculaire Mw compris entre 1700 et 3000 Daltons, caractérisées par une teneur en liaisons osidiques 1-6 (à la fois de type α et β) comprise entre 30 % et 45 %, une teneur en fibres indigestibles solubles comprise entre 75 % et 100 % (selon la méthode AOAC N°2001-03) et de remarquables propriétés hypoglycémiantes, qu'elles traduisent *in vitro* comme *in situ,* par un effet limitant vis-à-vis de la digestion de maltodextrines standard.

On connaît aussi la demande de brevet WO 2014/158777 qui décrit un carbohydrate utilisable comme ingrédient alimentaire (notamment en tant qu'agent réducteur de calories) contenant à la fois des oligomères linéaires et branchés, dont le taux de sucre est compris entre 5 % et 25 % en poids de son poids sec, avec une teneur en fibres comprise entre 10 % et 70 % de son poids sec. Une autre de ses caractéristiques est de présenter une teneur en polysaccharides avec des poids moléculaires élevés, telle que sa viscosité soit inférieure à 16 000 cPs, à 100 °F et à une teneur en matière sèche de 75 %.

On connaît également les demandes de brevet US 7,608,436 et WO 2008/085529 décrivant un produit alimentaire à base d'oligosaccharides, faiblement digestible. Lesdits oligosaccharides présentent ici une teneur en oligomères branchés supérieure à la teneur en oligomères linéaires, et une concentration en oligomères non linéaires et de degré de polymérisation supérieure ou égale à 3 supérieure à 20 % en poids sec.

On connaît enfin la demande de brevet WO 2014/145276 qui décrit des compositions à base de carbohydrates ayant un faible pouvoir calorique. Différentes familles de carbohydrates y sont décrites et revendiquées, notamment à travers leurs teneurs en molécules de degré de polymérisation 1 et 2, en liaisons 1-6, en somme des liaisons 1-4 et 1-6, à travers le ratio entre les teneurs en liaisons 1-4 et 1-6, et enfin à travers leur poids moléculaire.

La demande EP 0675137 A2 décrit quant à elle des glucans comprenant une structure cyclique d'au moins 14 liaisons alpha 1-4.

Par ailleurs, on connaît les produits commercialisés sous les noms de PROMITOR (Tate & Lyle), FIBERSOL (MATSUTANI), LITESSE (DUPONT DANISCO) et NUTRIOSE (ROQUETTE), qui sont tous des produits à base de polysaccharides, plus ou moins riches en fibres.

De ce qui précède, il résulte que des innovations continuelles ont été réalisées au moins ces 10 dernières années, dans le domaine des produits riches en fibres et susceptibles de présenter des avantages pour l'homme dans le cadre de nouveaux régimes alimentaires plus sains et mieux équilibrés. Il peut être notamment tout à fait intéressant de disposer d'un produit à la fois riche en fibres, et susceptible de ne libérer que très peu de glucose lorsqu'il est digéré par l'organisme ou alternativement de présenter une cinétique de libération du glucose très longue vis-à-vis de l'organisme. Ce faisant, cette dernière propriété apparaît potentiellement très intéressante dans le cadre de régimes pour patients diabétiques et/ou en vue de limiter les risques d'exposition face à cette pathologie.

### RESUME DE L'INVENTION

A cet égard, la société Demanderesse a poursuivi de nombreux travaux et des recherches laborieuses, qui lui ont permis de mettre au point des produits répondant à cette attente. Selon un mode de réalisation, les produits en question peuvent être définis comme des malto-oligo-saccharides, présentant notamment une teneur en liaisons α 1-4 comprise entre 70 % et 80 % du nombre total de liaisons 1-4 et une teneur en liaisons α 1-6 comprise entre 35 et 58% du nombre total de liaisons osidiques 1-6.

Comparativement aux produits de l'état de la technique connus de la Demanderesse, certains malto-oligo-saccharides de la présente Demande présentent des teneurs toute particulières en liaisons α 1-4 jamais décrites ou atteintes jusqu'alors. Parallèlement et selon les connaissances de la Demanderesse, il n'existe pas dans l'état de la technique de produits du type malto-oligo-saccharides présentant une teneur en liaisons α 1-4 comprise entre 70 % et 80 % du nombre total de liaisons 1-4.

En outre, c'est grâce à une approche originale associant les techniques de résonance magnétique nucléaire du proton (RMN 1H) et de chromatographie en phase gazeuse que la Demanderesse est parvenue à dégager l'influence du paramètre retenu (i.e. la teneur en liaisons α 1-4 relative à la totalité des liaisons 1-4) sur la teneur en fibres du produit final et la biodisponibilité du glucose vis-à-vis de l'organisme.

La RMN donne en effet accès aux proportions en liaisons osidiques totales en α 1-4 et α 1-6 d'une part, et aux autres liaisons osidiques d'autre part. Quant à la spectrométrie de masse, elle permet d'accéder aux proportions en liaisons osidiques totales en 1-4, 1-6, 1-2 et 1-3 mais ce, sans indication quant à la nature de l'anomérie. Cette dernière technique est mise en œuvre dans le cadre de la méthode bien connue de l'homme du métier dite de HAKOMORI, et permettant justement de quantifier les teneurs en liaisons osidiques totales en 1-4, 1-6, 1-2 et 1-3. Cette méthode décrite pour la première fois il y a plus de 50 ans (HAKOMORI, S., 1964, J. Biol. Chem., 55, 205) est aujourd'hui largement utilisée dans le monde scientifique (voir notamment la demande de brevet EP 1 006 128 déjà citée dans la présente Demande).

C'est sur la base de ces travaux et d'une telle approche que la Demanderesse a démontré la criticité de la teneur en liaisons α 1-4 relativement à la totalité des liaisons osidiques de type 1-4, en vue d'obtenir:
- un apport en fibres suffisant en vue de maintenir les effets bénéfiques précités liés à un régime riche en fibres,
- une biodisponibilité en glucose extrêmement faible, et notamment bien inférieure à celle affichée par la majorité des produits commerciaux disponibles à ce jour.

Cette biodisponibilité en glucose fait référence au taux de glucose libéré après digestion par les enzymes intestinales.

De manière tout à fait avantageuse, la régulation très précise de la teneur en liaisons α 1-4 relativement à la totalité des liaisons osidiques de type 1-4 dans l'intervalle précité est le principal levier pour aboutir au compromis idéal entre cet apport suffisant en fibres et cette biodisponibilité extrêmement faible en glucose.

De plus, c'est au moyen d'un procédé très simple que la Demanderesse est parvenue à synthétiser les malto-oligo-saccharides qui font l'objet de la présente invention. De manière très schématique, ce procédé consiste initialement à fournir une solution aqueuse de glucides riche en maltose, à ajouter au moins un polyol et au moins un acide, puis à réaliser un traitement thermique à haute température (entre 130°C et 300°C) sous pression réduite.

Enfin, il convient d'indiquer que dans toute la présente Demande, le taux de fibres, le taux de glucose libéré ou accessible après digestion enzymatique ainsi que les teneurs en liaisons osidiques sont déterminés selon des protocoles stricts qui font l'objet d'une description très détaillée dans la partie expérimentale relative à ladite Demande.

### DESCRIPTION DETAILLEE DE L'INVENTION

Un premier objet de la présente invention repose sur un procédé de fabrication des malto-oligosaccharides de l'invention comprenant les étapes consistant à :
a) fournir une solution aqueuse d'au moins 2 glucides, caractérisée en ce que 40 % à 95 % du poids sec de ladite solution est constitué de maltose,
b) mettre la solution aqueuse résultant de l'étape a) en présence d'au moins un polyol, et d'au moins un acide minéral ou organique,
c) augmenter éventuellement la teneur en matière sèche de la solution aqueuse résultant de l'étape b) jusqu'à au moins 75 % en poids de son poids total,
d) réaliser un traitement thermique sur la solution aqueuse résultant de l'étape b) ou éventuellement de l'étape c), à une température comprise entre 130°C et 300°C et sous une dépression comprise entre 50 et 500 mbars.

La première étape du procédé selon l'invention consiste à fournir une solution aqueuse de glucides, dont 40 % à 95 % de son poids sec est constitué de maltose.

De préférence, les autres glucides sont des composés saccharidiques de glucose. Ces glucides peuvent ainsi être fournis sous forme d'un ou plusieurs sirops de glucose.

Selon une variante de l'invention tout particulièrement préférée, la dite solution aqueuse résultant de l'étape a) contient en outre du glucose.

Dans cette étape, le maltose et les autres glucides peuvent être apportés sous forme de produits secs (poudres) ou alternativement sous forme liquide. S'il s'agit de produits secs, il convient de leur ajouter de l'eau de manière à réaliser la solution aqueuse objet de l'étape a). La forme liquide est appelée « sirop » et consiste en une solution aqueuse d'au moins un glucide. Une variante préférée de l'invention consiste à mélanger un sirop contenant au moins un glucide et au moins un glucide sous forme de produit sec. Selon cette variante, le mélange est facilité si on porte la température à au moins 50°C et au plus 90°C.

La solution aqueuse résultant de l'étape a) présente une teneur en matière sèche d'au moins 50 %, préférentiellement d'au moins 70 %, très préférentiellement d'au moins 80 % en poids de son poids total, et dans tous les cas d'au plus 95 % en poids de son poids total.

Un sirop d'au moins un glucide particulièrement préféré est un sirop dont la distribution des degrés de polymérisation (DP) est la suivante :
- de 1 % à 5 % de composés ayant un degré de polymérisation de 1
- de 40 % à 75 % de composés ayant un degré de polymérisation de 2
- de 10 % à 25 % de composés ayant un degré de polymérisation de 3
- de 5 % à 10 % de composés ayant un degré de polymérisation entre 4 inclus et 8 inclus
- de 1 % à 15 % de composés ayant un degré de polymérisation entre 9 inclus et 20 inclus
- de 1 % à 15 % de composés ayant un degré de polymérisation supérieur strictement à 20

chacun de ces % étant exprimé en % du poids total des glucides contenus dans ledit sirop, et la somme de ces % étant égale à 100 %.

Parmi les sirops les plus préférés, on peut citer le sirop commercialisé par la société Demanderesse sous le nom Sirop de glucose 5774, dont la répartition des degrés de polymérisation est concordante avec les gammes énumérées ci-dessus.

Un autre sirop d'au moins un glucide particulièrement préféré est un sirop dont la distribution des degrés de polymérisation (DP) est la suivante :
- de 8 % à 30 % de composés ayant un degré de polymérisation de 1
- de 40 % à 75 % de composés ayant un degré de polymérisation de 2
- de 7 % à 17 % de composés ayant un degré de polymérisation de 3
- de 3 % à 10 % de composés ayant un degré de polymérisation entre 4 inclus et 8 inclus
- de 0,1 % à 5 % de composés ayant un degré de polymérisation entre 9 inclus et 20 inclus
- de 0,1 % à 5 % de composés ayant un degré de polymérisation supérieur strictement à 20
chacun de ces % étant exprimé en % du poids total des glucides contenus dans ledit sirop, et la somme de ces % étant égale à 100 %.

Cet autre sirop peut notamment être obtenu en mélangeant le sirop de glucose 5774 avec du dextrose sous forme de poudre.

La deuxième étape du procédé selon l'invention consiste à mettre la solution aqueuse de glucides précédemment décrite en présence d'au moins un polyol, et d'au moins un acide minéral ou organique. Le mélange est facilité si on porte la température du milieu à au moins 50°C et au plus 90°C.

Le polyol mis en œuvre dans le procédé conforme à l'invention pourra notamment être choisi, sans pour autant que ce choix ne soit exhaustif, parmi le glycérol, l'érythritol, le xylitol, l'arabitol, le ribitol, le sorbitol, le dulcitol, le mannitol, le maltitol, l'isomaltitol, le lactitol et leurs mélanges, plus préférentiellement parmi le sorbitol, le mannitol et le maltitol, le polyol le plus préféré étant le maltitol. Le polyol représente 5 % à 30 %, préférentiellement 5 % à 25 %, très préférentiellement 5 % à 10 % en poids de la somme des poids secs de glucides, dudit polyol et de l'acide.

Le polyol est introduit sous forme d'une solution aqueuse, avec une teneur en matière sèche comprise entre 20 % et 90 %, préférentiellement entre 25 % et 85 %, et très préférentiellement entre 30 % et 80 % en poids de son poids total. Alternativement, le polyol peut être initialement sous forme anhydre ; dans ce cas, il peut être dissout directement par introduction dans le sirop contenant au moins un glucide, ou il peut être mis préalablement en solution aqueuse par dissolution dans l'eau.

Le procédé conforme à la présente invention met aussi en œuvre un acide minéral ou organique, préférentiellement organique, en tant que catalyseur de la réaction de polymérisation. Cet acide organique peut être choisi de manière non exhaustive parmi l'acide citrique, sulfurique, fumarique, succinique, gluconique, chlorhydrique, hydrochlorhydrique et les mélanges de ces acides, l'acide citrique étant le plus préféré.

Dans tous les cas, l'acide choisi ne devra pas présenter une volatilité trop importante, et ne devra pas présenter d'incompatibilité ou de points de vigilance à l'égard d'une future utilisation dans les domaines de la nutrition humaine et animale.

La quantité d'acide mise en œuvre est ici comprise entre 0,5 % et 2 %, préférentiellement entre 0,5 % et 1,5 %, et est très préférentiellement d'environ 1 % en poids dudit acide par rapport au poids sec de glucides, du polyol et dudit acide. Dans tous les cas, l'homme du métier saura adapter la quantité d'acide mise en œuvre, prenant notamment en considération les questions de neutralisation ultérieure, liée à l'emploi d'un éventuel excès dudit acide. L'acide en question peut être utilisé sous forme d'une solution aqueuse avec une teneur en matière sèche comprise entre 20 % et 90 %, préférentiellement entre 25 % et 85 %, et très préférentiellement entre 30 % et 80 % en poids de son poids total. Alternativement, ledit acide peut être initialement sous forme anhydre ; dans ce cas, il peut être dissout directement par introduction dans le sirop de glucides, ou il peut être mis préalablement en solution aqueuse par dissolution dans l'eau.

De manière préférée, l'homme du métier mettant en œuvre le procédé selon la présente invention cherchera à obtenir une teneur en matière sèche pour le milieu réactionnel incluant le mélange de glucides, le polyol et l'acide, comprise entre 20 % et 98 %, préférentiellement entre 25 % et 95 %, et très préférentiellement entre 30 % et 95 % en poids de son poids total. Il saura adapter cette teneur en matière sèche, notamment en fonction de la richesse souhaitée pour le mélange réactionnel, mais aussi entre autre en tenant compte de la viscosité du milieu (eu égard à d'éventuels problématiques de pompabilité et/ou transfert du milieu résultant). Il saura également l'adapter en vue de limiter ou même d'éviter, s'il le souhaite, l'étape c) optionnelle consistant à augmenter la teneur en matière sèche à hauteur d'au moins 75 % en poids sec de la solution aqueuse contenant les glucides, le polyol et l'acide.

La troisième étape du procédé selon l'invention est optionnelle puisqu'elle consiste, le cas échéant, à augmenter la teneur en matière sèche de la solution aqueuse résultant de l'étape b) jusqu'à au moins 75 % en poids de son poids total. Ceci est réalisé sous forme d'un traitement thermique, notamment à une température comprise entre 60°C et 150°C, préférentiellement entre 80°C et 120°C. De manière préférée, on appliquera une dépression comprise entre 50 mbars et 500 mbars, préférentiellement entre 100 mbars et 400 mbars. La durée de cette étape est comprise entre 4 et 20 heures. L'homme du métier saura adapter les paramètres temps, température et pression, notamment en fonction de sa teneur en matière sèche initiale et de la teneur en matière sèche qu'il souhaite obtenir au final. Pour l'Homme du métier, les termes « appliquer une dépression » signifie que la pression indiquée est inférieure à 1 bar en pression absolue, au contraire des termes « appliquer une pression » qui signifie que la pression est supérieure à la pression atmosphérique. En d'autres termes, lorsque l'on applique une dépression comprise entre X mbars et Y mbars, cela signifie que la pression absolue est comprise entre X mbars et Y mbars.

La quatrième étape du procédé selon l'invention consiste à réaliser un traitement thermique sur la solution aqueuse résultant de l'étape b) ou éventuellement de l'étape c), à une température comprise entre 130°C et 300°C et sous une dépression comprise entre 50 et 500 mbars. C'est sous ces conditions que s'effectue la réaction de polymérisation.

Cette étape est réalisée dans un réacteur de polymérisation, équipé de dispositifs de chauffe et permettant de travailler sous pression réduite. Un tel réacteur peut notamment consister en un four de polymérisation, ou un four sous vide. Alternativement, l'opération d'ajustement de la matière sèche et de polymérisation est réalisée dans le même réacteur, qui dispose avantageusement des moyens et dispositifs précités.

La réaction de polymérisation est conduite à une température comprise entre 130°C et 300°C, préférentiellement entre 150°C et 200°C. L'eau générée par la réaction est évacuée de manière continue par évaporation. Cette opération est conduite sous pression réduite, notamment à une dépression comprise entre 50 mbars et 500 mbars. Parallèlement, ladite réaction est conduite pendant un temps compris entre 5 minutes et 4 heures, préférentiellement entre 5 minutes et 2 heures.

La température et le temps de réaction sont des variables interdépendantes. Il conviendra de veiller à ne pas élever trop la température de manière à éviter tout phénomène de pyrolyse et/ou de dégradation thermique des produits (une telle dégradation pouvant altérer les propriétés sensorielles du produit alimentaire fabriqué au final). Néanmoins, le temps de réaction diminue d'autant que la température augmente, en vue d'une polymérisation complète. De ce point de vue, les produits selon la présente invention peuvent tout à fait bien être fabriqués à une température de l'ordre de 250°C et avec un temps de séjour de 10 minutes, qu'à une température d'environ 180°C et un temps de séjour d'environ 90 minutes. En utilisant le procédé de l'invention, l'homme du métier peut faire varier la teneur en liaisons α 1-4 du nombre total de liaisons osidiques 1-4 selon la manière suivante; plus la réaction de polymérisation avance, plus cette teneur diminue.

Un deuxième objet de la présente invention consiste en des malto-oligo-saccharides susceptibles d'être obtenus selon le procédé défini précédemment.

Ces malto-oligo saccharides présentent une teneur en liaisons α 1-4 comprise entre 70 % et 80 % du nombre total de liaisons osidiques 1-4. Ces malto-oligo saccharides présentent en outre une teneur en liaisons α 1-6 comprise entre 35 et 58% du nombre total de liaisons osidiques 1-6.

Ces malto-oligo-saccharides, objets de la présente invention peuvent également être caractérisés en ce qu'ils présentent une teneur en liaisons α 1-6 comprise entre 38 et 52% du nombre total de liaisons osidiques 1-6, préférentiellement entre 40 % et 50 % du nombre total de liaisons osidiques 1-6.

Ces malto-oligo-saccharides peuvent également être caractérisés en ce qu'ils présentent une teneur en fibres comprise entre 50 % et 70 %.

Ces malto-oligo-saccharides sont également caractérisés en ce qu'ils présentent un taux de glucose libéré ou accessible après digestion enzymatique compris entre 1 % et 12 %, plus préférentiellement entre 3 et 9%.

Les malto-oligo saccharides de l'invention peuvent présenter un ratio du nombre total de liaisons osidiques 1-4 sur le nombre total de liaisons osidiques 1-6 supérieur à 1.

De préférence, les malto-oligo saccharides de l'invention présentent un ratio du nombre total de liaisons 1-4sur le nombre total de liaisons 1-6 allant de 1,03 à 2,50, par exemple de 1,05 à 2.

Un dernier objet de la présente invention concerne l'utilisation des malto-oligo-saccharides selon l'invention ou susceptibles d'être obtenus selon procédé défini précédemment en nutrition humaine et animale.

A titre non limitatif, les malto-oligo-saccharides selon l'invention ou susceptibles d'être obtenus selon procédé défini précédemment peuvent être incorporés dans des compositions ou produits destinés à l'ingestion et à l'administration orale tels que diverses denrées alimentaires comme les confiseries, les pâtisseries, les crèmes glacées, les pâtes à mâcher, les chewing-gums, les boissons, les confitures, les soupes, les préparations à base de lait, les yaourts, les gâteaux, les aliments préparés pour animaux, les compléments alimentaires, les produits pharmaceutiques, vétérinaires, diététiques ou hygiéniques tels que par exemple les élixirs, les sirops contre la toux, les tablettes ou les comprimés, les pastilles, les solutions d'hygiène buccale, les pâtes et les gels dentifrices.

Les exemples qui suivent permettront de mieux appréhender la présente invention, sans pour autant en limiter la portée.

### EXEMPLES

### METHODES EXPERIMENTALES

Dans toute la présente Demande, les teneurs en liaisons osidiques sont déterminées par RMN, et par la méthode de HAKOMORI.

La RMN permet d'accéder aux proportions en liaisons alpha 1,4 et alpha 1-6 d'une part, et aux autres liaisons osidiques d'autre part.

La méthode d'HAKOMORI permet d'accéder aux teneurs en liaisons osidiques totales en 1-4, 1-6, 1-2 et 1-3.

En ce qui concerne la RMN, on utilise un spectromètre à transformée de Fourier Avance III (Bruker Spectrospin), opérant à 400MHz, et utilisant des tubes RMN de 5 mm, à 60°C. De manière plus générale, on peut utiliser tout autre spectromètre à transformée de Fourier, pour peu que ledit spectromètre soit équipé avec tous les accessoires permettant la réalisation et l'exploitation d'un spectre du proton, ainsi que d'un accessoire permettant de travailler à des températures supérieures à la température ambiante. On utilise de l'eau deutériée, ou D₂O, (min 99 %), Euryso Top (groupe CEA, Gif-sur-Yvette, France) et du sel sodique de l'acide 3-triméthylsilyl-1-propane sulfonique, ou TSPSA (Aldrich, réf 178837).

Le mode opératoire des expériences est le suivant :
- Introduire 10 mg d'échantillon et 0,75 mL de D₂0 dans un tube RMN.
- Boucher le tube, mélanger, puis placer dans un bain-marie.
- Après dissolution, retirer le tube du bain-marie et laisser refroidir à température ambiante.
- Ajouter 50 µL d'une solution de TSPSA à 10 mg/g dans D₂O.
- Adapter le spinner sur le tube et placer le tout dans l'aimant.
- Effectuer l'acquisition, sans suppression de solvant, avec un temps de relaxation d'au moins 10 s et sans rotation, après les réglages appropriés de l'instrument (field, lock phase et shims) Utiliser une fenêtre spectrale comprise entre au moins -0.1 ppm et 9 ppm, en se référant au signal des méthyles du TSPSA calibré à 0 ppm.

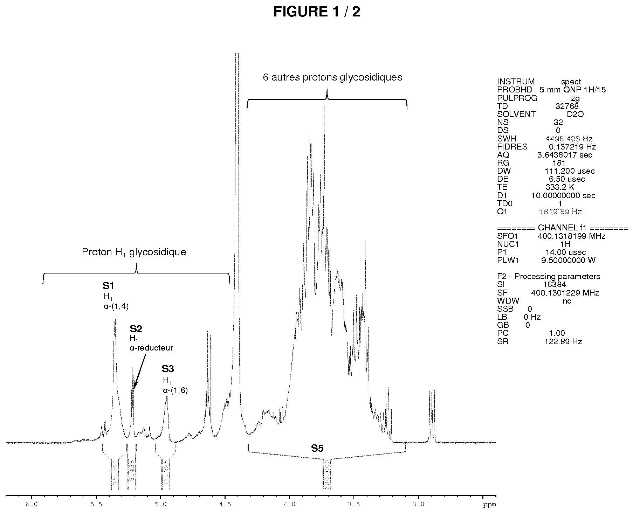

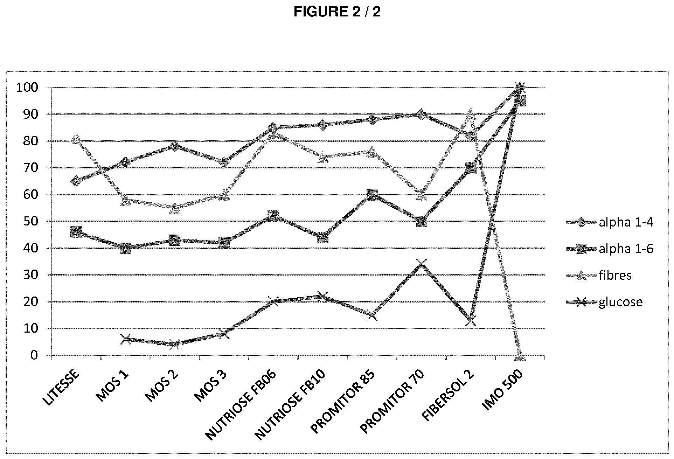

Le spectre est exploité après transformation de Fourier, correction de phase et soustraction de la ligne de base en mode manuel (sans multiplication exponentielle, LB=GB=0). Les résultats sont exploités de la manière suivante :
- Intégrer les signaux ; on pourra notamment se référer à la figure 1 / 2 pour les bornes d'intégration.
- Normaliser à 600 le signal S5 correspondant aux protons non échangeables d'une unité anhydroglucose (H₂, H₃, H₄, H₅ et 2H₆) ; le reste du signal correspondant à l'ensemble des protons H₁ (liaisons et terminaisons réductrices).
- Relever les valeurs de S1 (H₁ alpha (1,4), S2 (H₁ alpha réducteur) et S3 (H₁ alpha (1,6)).
- Déterminer les bêta-réducteurs S4 en réalisant l'opération S2*0.6/0,4.
- Calculer S6 en réalisant l'opération S6 = 100 - (S1 +S2+S3+S4)
- Déterminer les proportions de liaison alpha-(1,4), alpha-(1,6) et autres liaisons, en faisant la somme des 3 surfaces respectives (S1, S3 et S6) et en les normalisant à 100 pour les exprimer en % (soit %i = Si*100/(S1 +S3+S6)).

**Tableau 1**

| Surface intégrée | Bornes d'intégration (en ppm) | | Types de liaisons |
|---|---|---|---|
| S1 | 5.45 | 5.26 | H₁ α-(1,4) |
| S2 | 5.26 | 5.19 | H₁ α-réducteurs |
| S3 | 5.04 | 4.88 | H₁ α-(1,6) |
| S5 | 4.32 | 3.10 | Autres Protons (H₂, H₃, H4 etc ...) soit 6 protons |

La méthode de HAKOMORI est celle décrite dans la publication de 1964, J. Biol. Chem., 55, 205.

Dans toute la présente Demande, le taux de glucose libéré ou accessible après digestion enzymatique est déterminé selon la méthode suivante.
- Peser 0,3 g sec de produit à tester.
- Ajouter 75 ml de tampon maléate de Na pH 7,00 à 0,1 mol/l (Fluka, référence 63180).
- Agiter jusqu'à la dissolution du produit.
- Placer les flacons au bain marie pendant 15 minutes, pour que la température de la solution soit de 37°C.
- Prélever 0,75 ml de la solution initiale et ajouter 0,075 g de pancréatine de porc après le prélèvement de la solution initiale (Sigma, référence P7545) ; cette opération correspond à l'origine des temps
- Incuber à 37°C au bain thermostaté sous agitation pendant 30 minutes.
- Réaliser un prélèvement de 0,75 ml.
- Ajouter 0,40 g de muqueuse intestinale de rat (Sigma, référence 11630).
- Incuber pendant 3h30 à 37°C au bain thermostaté sous agitation.
- Réaliser pendant ces 3h30 des prélèvements de 0,75 ml aux temps 60, 120, 180 et 240 minutes.
- Arrêter la réaction enzymatique en plaçant les prélèvements dans un bain à sec à 100°C, pendant 10 minutes.
- Réaliser le dosage du glucose des prélèvements (méthode enzymatique standard GOD).
- Calculer le taux de glucose libéré lors de la digestion du produit à l'issue des 3h30 (exprimé en %) : concentration en glucose en g/L du prélèvement ^{∗} (100/matière sèche du produit) ^{∗} (volume du digestat en ml/1000) ^{∗} (100/poids du produit humide en g).

Dans toute la présente Demande, le taux de fibres est mesuré selon la méthode AOAC N° 2001-03.

### PRODUITS SELON L'INVENTION

Dans les essais n° 1 à 5, on a réalisé 5 produits selon le procédé conforme à la présente invention.

On dispose d'un sirop de glucose 5774 (« Flolys D57 ») commercialisé par la société ROQUETTE à 85 % de matière sèche.

Ce sirop est dilué à 50°Bx.

On prépare 1 kilo de matière avec les % massiques mentionnés dans le tableau ci-dessous, dans un bêcher en verre. Ledit bêcher est placé sur une plaque chauffante, sous agitation avec un barreau aimanté réglé à 500 tpm, la température étant réglée à 60°C.

Une fois cette température atteinte, on introduit, sous forme de poudre, dans le bêcher le glucose commercialisé sous le nom de Dextrose Anhydre C, par la société ROQUETTE.

On ajoute ensuite à cette solution aqueuse sous forme poudre les produits suivants :
- le maltitol commercialisé sous le nom SWEETPEARL P200 par la société ROQUETTE
- L'acide citrique commercialisé par la société SIGMA, de pureté supérieure ou égale à 99,5 %

Les % massiques des constituants sont donnés dans le tableau 2, pour les essais n°1, 2, 3, 4 et 5 qui représentent l'invention.

**Tableau 2**

| Essai n° | 1 | 2 | 3 | **4** | **5** |
|---|---|---|---|---|---|
| Flolys D57 (85 % de MS) | 81 | 68 | 68 | 72 | 71 |
| Glucose | 9 | 22.2 | 22.2 | 18 | 18 |
| Maltitol | 9 | 8.9 | 8.9 | 9 | 9 |
| Acide citrique | 1 | 0.9 | 0.9 | 1 | 2 |

Après dissolution complète des poudres, soit quelques minutes, le mélange est limpide.

On prélève alors 120 grammes du mélange qui sont transférés dans une barquette en aluminium commercialisé par la société PRO'JET sous la référence KPL1001.

Les barquettes sont placées dans une étuve sous vide pendant 20 heures à 80°C puis 6 heures à 120 °C. Une dépression de 125 mbars est appliquée dans l'étuve. On obtient alors une matière sèche de 95,0 %.

Les barquettes avec le produit sec sont alors placées dans une seconde étuve préalablement chauffée à 200°C, l'ensemble étant mis sous une dépression de 125 mbars. On retire les barquettes 90 minutes plus tard.

Le produit est alors dilué avec de l'eau à 30 % de matière sèche.

Pour chacun des 5 essais précédents, la Demanderesse a déterminé :
- le % en liaisons osidiques alpha 1-4 du nombre total de liaisons osidiques 1-4
- le % en liaisons osidiques alpha 1-6 du nombre total de liaisons osidiques 1-6
- le ratio du nombre total de liaisons osidiques 1-4 sur le nombre total de liaisons osidiques 1-6
- la teneur en fibres en %
- le taux de glucose libéré en %

### PRODUITS HORS INVENTION

La Demanderesse a également déterminé ces mêmes paramètres pour les produits suivants :
- NUTRIOSE FB06 commercialisé par la société Demanderesse (essai n°4)
- NUTRIOSE FB10 commercialisé par la société Demanderesse (essai n°5)
- LITESSE ULTRA commercialisé par la société DUPONT DANISCO (essai n°6)
- PROMITOR 70 commercialisé par la société TATE & LYLE (essai n°7)
- PROMITOR 85 commercialisé par la société TATE & LYLE (essai n°8)
- FIBERSOL 2 commercialisé par la société MATSUTANI (essai n°9)
- IMO 500 commercialisé par la société (essai n° 10)

L'ensemble des résultats a été rapporté dans le tableau 3, de même que sur la figure 2 / 2, à l'exception des produits MOS 4 et 5.

**Tableau 3**

| Produit | alpha 1-4 (%) | alpha 1-6 (%) | ratio 1-4/1-6 | fibres (%) | glucose (%) |
|---|---|---|---|---|---|
| LITESSE | 67 | 53 | 0,61 | 81 | --- |
| MAL TO-OLIGO-SACCHARIDE 1 (MOS 1) | 72 | 40 | 2,11 | 58 | 6 |
| MAL TO-OLIGO-SACCHARIDE 2 (MOS 2) | 78 | 43 | 1,37 | 55 | 4 |
| MAL TO-OLIGO-SACCHARIDE 3 (MOS 3) | 72 | 42 | 1,10 | 60 | 8 |
| MAL TO-OLIGO-SACCHARIDE 4 (MOS 4) | 66 | 42 | 1,45 | 65 | --- |
| MAL TO-OLIGO-SACCHARIDE 5 (MOS 5) | 81 | 56 | 1,40 | 62 | --- |
| NUTRIOSE FB06 | 85 | 52 | 2,06 | 83 | 20 |
| NUTRIOSE FB10 | 86 | 44 | 1,75 | 74 | 22 |
| PROMITOR 85 | 88 | 60 | 1,15 | 76 | 15 |
| PROMITOR 70 | 90 | 50 | 1,73 | 60 | 34 |
| FIBERSOL 2 | 82 | 70 | 2,11 | 90 | 13 |
| IM0 500 | 100 | 95 | 1,34 | 0 | 100 |

On peut constater de manière remarquable que tous les produits commerciaux appartenant à l'état de la technique et riches en fibres, présentent une teneur en liaisons osidiques alpha 1-4 supérieure à 80 %, celle-ci étant comprise dans un intervalle très restreint, entre 80 % et 90 %.

Par ailleurs, l'ensemble de ces produits présente une teneur en fibres d'au moins 60 %, dans la plupart des cas d'au moins 75% et une teneur en glucose disponible systématiquement supérieure à 10 %, dans la plupart des cas supérieure à 15 %, et même parfois supérieure à 20 %.

A l'inverse, les 5 produits selon l'invention possèdent une teneur en liaisons osidiques α 1-4 comprise entre 66 % et 81 %. On constate que, de manière particulièrement avantageuse, ces produits possèdent une richesse en fibres importante, puisque celle-ci se situe aux alentours de 60 %. Enfin, 3 produits selon l'invention présentent une très faible teneur en glucose disponible, à des niveaux encore jamais atteints dans le passé. Ceci est particulièrement surprenant du fait que ces nouveaux produits, bien que riches en fibres, présentent une teneur en ces fibres inférieure à celle des autres produits testés. Aussi, seuls ces produits présentent le compromis idéal entre un produit relativement riche en fibres, et donc parfaitement adapté dans des régimes alimentaires sains et équilibrés, et un produit avec une très faible biodisponibilité du glucose vis-à-vis de l'organisme, donc parfaitement adapté à des patients diabétiques ou dans des régimes visant à diminuer la sensibilité à cette pathologie.

## Revendications

1. Procédé de fabrication de malto-oligo-saccharides comprenant les étapes consistant à :
a) fournir une solution aqueuse d'au moins 2 glucides, **caractérisée en ce que** 40 % à 95 % du poids sec de ladite solution est constitué de maltose,
b) mettre la solution aqueuse résultant de l'étape a) en présence d'au moins un polyol, et d'au moins un acide minéral ou organique,
c) augmenter éventuellement la teneur en matière sèche de la solution aqueuse résultant de l'étape b) jusqu'à au moins 75 % en poids de son poids total,
d) réaliser un traitement thermique sur la solution aqueuse résultant de l'étape b) ou éventuellement de l'étape c), à une température comprise entre 140°C et 300°C et sous une dépression comprise entre 50 et 500 mbars,
lesdits malto-oligo-saccharides étant des saccharides comprenant au moins 2 unités saccharides et ces malto-oligo-saccharides présentant une teneur en liaisons α 1-4 comprise entre 70 % et 80 % du nombre total de liaisons osidiques 1-4 et une teneur en liaisons α 1-6 comprise entre 35 et 58% du nombre total de liaisons osidiques 1-6.

2. Procédé selon la revendication 1, **caractérisé en ce que** la solution aqueuse résultant de l'étape a) contient du glucose.

3. Procédé selon la revendication 1 ou 2, **caractérisé en ce que** la solution aqueuse résultant de l'étape a) présente une teneur en matière sèche d'au moins 50 %, préférentiellement d'au moins 70 %, très préférentiellement d'au moins 80 % en poids de son poids total, et dans tous les cas d'au plus 95 % en poids de son poids total.

4. Procédé selon l'une quelconque des revendications 1 à 3, **caractérisé en ce que** le polyol est choisi parmi le glycérol, l'érythritol, le xylitol, l'arabitol, le ribitol, le sorbitol, le dulcitol, le mannitol, le maltitol, l'isomaltitol, le lactitol et leurs mélanges, plus préférentiellement parmi le sorbitol, le mannitol et le maltitol, le polyol le plus préféré étant le maltitol.

5. Procédé selon l'une quelconque des revendications 1 à 4, **caractérisé en ce que** l'acide lorsqu'il est organique est choisi parmi l'acide citrique, sulfurique, fumarique, succinique, gluconique, chlorhydrique, hydrochlorhydrique et les mélanges de ces acides, l'acide citrique étant le plus préféré.

6. Procédé selon l'une quelconque des revendications 1 à 5, **caractérisé en ce que** l'étape c) est réalisée sous forme d'un traitement thermique, à une température comprise entre 60°C et 150°C, préférentiellement entre 80°C et 120°C.

7. Procédé selon la revendication 6, **caractérisé en ce que** l'étape c) est réalisée sous une dépression comprise entre 50 mbars et 500 mbars, préférentiellement entre 100 mbars et 400 mbars.

8. Procédé selon l'une quelconque des revendications 1 à 7, **caractérisé en ce que** l'étape d) est réalisée entre 150°C et 200°C.

9. Procédé selon la revendication 8, **caractérisé en ce que** l'étape d) est réalisée à une pression comprise entre 50 mbars et 500 mbars.

10. Malto-oligo-saccharides susceptibles d'être obtenus selon le procédé tel que revendiqué dans l'une quelconque des revendications 1 à 9.

11. Malto-oligo-saccharides selon la revendication 10, **caractérisé en ce qu'**ils présentent une teneur en liaisons α 1-6 comprise entre 38 et 52% du nombre total de liaisons osidiques 1-6, préférentiellement une teneur en liaisons α 1-6 comprise entre 40 % et 50 % du nombre total de liaisons osidiques 1-6.

12. Malto-oligo-saccharides selon l'une quelconque des revendications 10 ou 11, **caractérisés en ce qu'**ils présentent une teneur en fibres comprise entre 50 % et 70 %.

13. Malto-oligo-saccharides selon l'une quelconque des revendications 10 à 12, **caractérisés en ce qu'**ils présentent un taux de glucose libéré ou accessible après digestion enzymatique compris entre 1 % et 12 %, plus préférentiellement entre 3 et 9%.

14. Utilisation des malto-oligo-saccharides selon l'une quelconque des revendications 10 à 13 en nutrition humaine et animale.

## Patentansprüche

1. Verfahren zur Herstellung von Malto-Oligo-Sacchariden, umfassend die Schritte :
a) Bereitstellen einer wässrigen Lösung von mindestens zwei Kohlenhydraten, **dadurch gekennzeichnet, dass** 40 bis 95 % des Trockengewichts dieser Lösung aus Maltose besteht,
b) Einbringen der aus Schritt a) resultierenden wässrigen Lösung in Gegenwart von mindestens einem Polyol und mindestens einer mineralischen oder organischen Säure,
c) gegebenenfalls Erhöhen des Trockensubstanzgehalts der aus Schritt b) resultierenden wässrigen Lösung auf mindestens 75 Gew.-% ihres Gesamtgewichts,
d) Durchführen einer Wärmebehandlung der aus Schritt b) oder gegebenenfalls aus Schritt c) resultierenden wässrigen Lösung bei einer Temperatur zwischen 140°C und 300°C und bei einem Unterdruck von zwischen 50 und 500 mbar,
wobei die Malto-Oligo-Saccharide Saccharide sind, die mindestens zwei Saccharid-Einheiten umfassen, und diese Malto-Oligo-Saccharide einen Gehalt α 1-4 Bindungen zwischen 70% und 80% der Gesamtzahl der 1-4 ossidischen Bindungen und einen Gehalt α 1-6 Bindungen zwischen 35 und 58% der Gesamtzahl der 1-6 ossidischen Bindungen aufweisen.

2. Verfahren nach Anspruch 1, **dadurch gekennzeichnet, dass** die aus Schritt a) resultierende wässrige Lösung Glucose enthält.

3. Verfahren nach Anspruch 1 oder 2, **dadurch gekennzeichnet, dass** die aus Schritt a) resultierende wässrige Lösung einen Trockensubstanzgehalt von mindestens 50 Gew.-%, bevorzugt von mindestens 70 Gew.-%, stark bevorzugt von mindestens 80 Gew.-% ihres Gesamtgewichts und in jedem Fall von höchstens 95 Gew.-% ihres Gesamtgewichts aufweist.

4. Verfahren nach einem der Ansprüche 1 bis 3, **dadurch gekennzeichnet, dass** das Polyol gewählt ist aus Glycerin, Erythrit, Xylit, Arabit, Ribit, Sorbit, Dulcit, Mannit, Maltit, Isomaltit, Lactit und deren Mischungen, bevorzugter aus Sorbit, Mannit und Maltit, wobei das am stärksten bevorzugte Polyol Maltit ist.

5. Verfahren nach einem der Ansprüche 1 bis 4, **dadurch gekennzeichnet, dass** die Säure, wenn sie organisch ist, gewählt ist aus Zitronen-, Schwefel-, Fumar-, Bernstein-, Glucon-, Chlorwasserstoff- und Salzsäure und Mischungen dieser Säuren, wobei Zitronensäure am stärksten bevorzugt wird.

6. Verfahren nach einem der Ansprüche 1 bis 5, **dadurch gekennzeichnet, dass** Schritt c) in Form einer Wärmebehandlung bei einer Temperatur zwischen 60°C und 150°C, bevorzugt zwischen 80°C und 120°C, durchgeführt wird.

7. Verfahren nach Anspruch 6, **dadurch gekennzeichnet, dass** Schritt c) bei einem Unterdruck von zwischen 50 mbar und 500 mbar, bevorzugt zwischen 100 mbar und 400 mbar, durchgeführt wird.

8. Verfahren nach einem der Ansprüche 1 bis 7, **dadurch gekennzeichnet, dass** Schritt d) bei zwischen 150°C und 200°C durchgeführt wird.

9. Verfahren nach Anspruch 8, **dadurch gekennzeichnet, dass** Schritt d) bei einem Druck zwischen 50 mbar und 500 mbar durchgeführt wird.

10. Malto-Oligo-Saccharide, welche nach dem in einem der Ansprüche 1 bis 9 beanspruchten Verfahren erhalten werden können.

11. Malto-Oligo-Saccharide nach Anspruch 10, **dadurch gekennzeichnet, dass** sie einen Gehalt α 1-6 Bindungen zwischen 38 und 52% der Gesamtzahl der 1-6 ossidischen Bindungen, bevorzugt einen Gehalt α 1-6 Bindungen zwischen 40% und 50% der Gesamtzahl der 1-6 ossidischen Bindungen, aufweisen.

12. Malto-Oligo-Saccharide nach einem der Ansprüche 10 oder 11, **dadurch gekennzeichnet, dass** sie einen Fasergehalt zwischen 50% und 70% aufweisen.

13. Malto-Oligo-Saccharide nach einem der Ansprüche 10 bis 12, **dadurch gekennzeichnet, dass** sie einen Gehalt an freigesetzter oder nach enzymatischer Verdauung zugänglicher Glukose von zwischen 1% und 12%, stärker bevorzugt zwischen 3 und 9%, aufweisen.

14. Verwendung der Malto-Oligo-Saccharide nach einem der Ansprüche 10 bis 13 in der Human- und Tierernährung.

## Claims

1. A method for manufacturing maltooligosaccharides comprising the steps consisting in:
a) providing an aqueous solution of at least two carbohydrates, **characterized in that** 40% to 95% of the dry weight of said solution consists of maltose,
b) placing the aqueous solution resulting from step a) in contact with at least one polyol and at least one inorganic or organic acid,
c) optionally increasing the solids content of the aqueous solution resulting from step b) up to at least 75% by weight of the total weight thereof,
d) carrying out a heat treatment on the aqueous solution resulting from step b) or optionally from step c), at a temperature of between 140°C and 300°C under a negative pressure of between 50 and 500 mbar,
said maltooligosaccharides being saccharides comprising at least 2 saccharide units and having a content of α-1,4- bonds of between 70% and 80% of the total number of 1,4-glycosidic bonds and a content of α-1,6- bonds of between 35% and 58% of the total number of 1,6-glycosidic bonds.

2. The method as claimed in claim 1, **characterized in that** the aqueous solution resulting from step a) contains glucose.

3. The method as claimed in claim 1 or 2, **characterized in that** the aqueous solution resulting from step a) has a solids content of at least 50%, preferably of at least 70%, very preferably of at least 80% by weight of the total weight thereof, and in any case of at most 95% by weight of the total weight thereof.

4. The method as claimed in any one of claims 1 to 3, **characterized in that** the polyol is selected from glycerol, erythritol, xylitol, arabitol, ribitol, sorbitol, dulcitol, mannitol, maltitol, isomaltitol, lactitol and mixtures thereof, more preferably from sorbitol, mannitol and maltitol, the most preferred polyol being maltitol.

5. The method as claimed in any one of claims 1 to 4, **characterized in that**, when the acid is organic, it is selected from citric, sulfuric, fumaric, succinic, gluconic and hydrochloric acid and mixtures of these acids, citric acid being the most preferred.

6. The method as claimed in any one of claims 1 to 5, **characterized in that** step c) is carried out in the form of a heat treatment at a temperature of between 60°C and 150°C, preferably between 80°C and 120°C.

7. The method as claimed in claim 6, **characterized in that** step c) is carried out under a negative pressure of between 50 mbar and 500 mbar, preferably between 100 mbar and 400 mbar.

8. The method as claimed in any one of claims 1 to 7, **characterized in that** step d) is carried out at between 150°C and 200°C.

9. The method as claimed in claim 8, **characterized in that** step d) is carried out at a pressure of between 50 mbar and 500 mbar.

10. Maltooligosaccharides able to be obtained according to the method as claimed in any one of claims 1 to 9.

11. The maltooligosaccharides as claimed in claim 10, **characterized in that** they have a content of α-1,6- bonds of between 38% and 52% of the total number of 1,6-glycosidic bonds, and preferably a content of α-1,6- bonds of between 40% and 50% of the total number of 1,6-glycosidic bonds.

12. The maltooligosaccharides as claimed in either one of claims 10 and 11, **characterized in that** they have a fiber content of between 50% and 70%.

13. The maltooligosaccharides as claimed in any one of claims 10 to 12, **characterized in that** they have an amount of glucose released or accessible after enzymatic digestion of between 1% and 12%, more preferably of between 3% and 9%.

14. The use of the maltooligosaccharides as claimed in any one of claims 10 to 13 in human food and animal feed.
